# FASCICULE DE BREVET EUROPEEN

(11) **EP 0 767 166 B1**
(45) Date de publication et mention de la délivrance du brevet: **31.05.2000**
(21) Numéro de dépôt: 96402083.8
(22) Date de dépôt: 01.10.1996
(51) Int. Cl.: C07C 263/12, C07C 265/16

(54) **Procédé de préparation d'isocyanates d'acyle**
Verfahren zur Herstellung von Acylisocyanaten
Process for the preparation of acyl isocyanates

(30) Priorité: 05.10.1995 FR 9511722
(43) Date de publication de la demande: 09.04.1997
(73) Titulaire: SOCIETE NATIONALE DES POUDRES ET EXPLOSIFS, F-75181 Paris Cédex 04 (FR)
(72) Inventeur: Bertrand, Guy, 31320 Pechbusque (FR); Guyot, Daniel, 31330 Montastruc la Conseillere (FR); Denarie, Michel, 84210 Pernes les Fontaines (FR); Senet, Jean-Pierre, Herbeauvilliers, 77760 Buthiers (FR)
(74) Mandataire: Pech, Bernard

(56) Documents cités:
- DATABASE WPI Week 7712 Derwent Publications Ltd., London, GB; AN 77-21324y XP002004594 & SU-A-498 290 (TKACHEV A S) , 14 Septembre 1976
- CHEMICAL ABSTRACTS, vol. 100, no. 7, 1984 Columbus, Ohio, US; abstract no. 51682q, XP002004593 & ZH. OBSHCH. KHIM., vol. 53, no. 9, 1983, pages 2155-2156, V.P. KOZYUKOV ET AL:
- H. HAGEMANN: "Methoden Der Organischen Chemie (Houben-Weyl) Band E4, Kohlensäurederivate" 1983 , GEORG THIEME VERLAG , STUTTGART . NEW YORK XP002004592 * page 806 - page 808 *
- JERRY MARCH: "Advanced Organic Chemistry 3rd Edition" , 01-01-1985, JOHN WILEY & SONS, NEW YORK
- ZEILER: "The chemistry of phosgène", CHEMICAL REVIEWS, , 28-07-1972, Vol. 73, no. 1, pages 75 à 83

## Description

La présente invention concerne un nouveau procédé de préparation d'isocyanates d'acyle au moyen de chlorure d'oxalyle.

Les isocyanates d'acyle sont, en raison de leur excellente réactivité, des intermédiaires très utiles dans le domaine de l'agrochimie et de la pharmacie, notamment pour former des urées et des carbamates.

Le premier procédé de préparation des isocyanates d'acyle consistait à faire réagir un chlorure d'acyle avec le cyanate d'argent (J. Am. Chem. Soc. 62, 1595 (1940). Malheureusement ce procédé ne peut être utilisé industriellement en raison du coût excessif du cyanate d'argent.

Selon un autre procédé, on remplace le cyanate d'argent par de l'acide isocyanique (brevet US n° 3 155 700). Mais cet acide est très instable et très difficile à préparer par décomposition de l'acide isocyanurique à une température très élevée telle que 620°C.

De 1962 à 1965, A.J. Speziale et al ont développé un procédé de préparation d'isocyanates d'acyle dans lequel des amides sont mis à réagir avec du chlorure d'oxalyle (J. Org. Chem., 27, 3742 (1962), 28, 1805 (1963), 30, 4306 (1965). Malheureusement ce procédé donne des résultats très variables selon les amides de départ utilisés, en particulier les rendements en isocyanates d'acyle à partir d'amides aliphatiques légères primaires ou secondaires non substitués sur le carbone en α par un groupe électro-attracteur sont très faibles. De plus la grande quantité d'acide chlorhydrique formée en même temps que les isocyanates détériore les installations et empêche l'obtention des isocyanates sensibles aux conditions acides.

Dans l'article du Chemical Abstracts, vol.100, n°7, 1984, n°51682q, il est décrit la synthèse de quelques isocyanates d'acyle par réaction du phosgène sur des N,N-bis(trialkylsilyl)carboxamides. Seuls des isocyanates d'acyle aliphatiques ont été préparés selon ce procédé. Les rendements sont faibles, de l'ordre de 14 % à 59 %. De plus, ce procédé est délicat à mettre en oeuvre car le phosgène est un composé très toxique qu'il faut manipuler avec précaution.

La présente invention a pour objet un procédé de préparation d'isocyanates d'acyle qui ne présente pas les inconvénients des procédés antérieurs et qui permet l'obtention d'une large variété d'isocyanates d'acyle dans des conditions réactionnelles simplifiées.

Selon la présente invention, le procédé de préparation d'isocyanates d'acyle est caractérisé en ce qu'on fait réagir le chlorure d'oxalyle avec un N-trialkylsilylcarboxamide ou un N,N-bis(trialkylsilyl)carboxamide.

Le schéma réactionnel du procédé est le suivant : 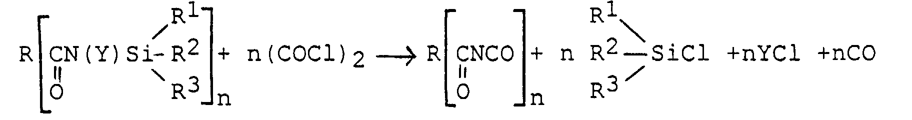 dans lequel R représente le radical de l'amide, R¹, R² et R³, identiques ou différents, représentent un radical alkyle, Y représente un atome d'hydrogène ou un groupe 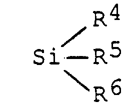 dans lequel R⁴, R⁵ et R⁶, identiques ou différents, représentent un radical alkyle
et n représente le nombre 1 ou 2.

Le procédé selon l'invention permet l'obtention des isocyanates d'acyle et en particulier des isocyanates d'acyle dérivés d'amides aliphatiques, avec un bon rendement. En même temps que l'isocyanate d'acyle, il se forme un trialkylchlorosilane. Celui-ci peut être complètement recyclé pour préparer l'amide silylé de départ, ce qui est un grand avantage. De plus, de l'acide chlorhydrique n'est pas libéré lorsque le composé de départ est un carboxamide disilylé et, lorsque le carboxamide est monosilylé, la quantité d'acide chlorhydrique qui se forme est inférieure de moitié à celle libérée dans les procédés antérieurs.

Les N-trialkylsilylcarboxamides ou les N,N-bis(trialkylsilyl)carboxamides utilisés comme composés de départ sont des composés disponibles dans le commerce ou qui peuvent se préparer très facilement selon le procédé décrit par J.F. Klebe et al dans J. Am. Chem. Soc. (1966), 88, 3390-95, notamment par réaction des trialkylchlorosilanes avec les carboxamides.

Le procédé selon l'invention convient en particulier pour la transformation des carboxamides silylés de formule 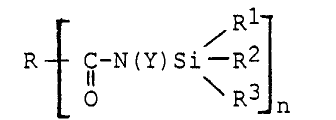 dans laquelle :
R représente un radical - aliphatique linéaire ou ramifié, en C₁ à C₂₀, de préférence en C₁ à C₁₀, saturé ou non, substitué ou non,
   - cycloaliphatique en C₄ à C₇, saturé ou non, substitué ou non,
   - phényle, phénylène, naphtyle, naphtylène, substitué ou non,
   - hétéroaromatique, de préférence à 5 ou 6 chaînons, substitué ou non, le ou les hétéroatomes étant choisis de préférence dans le groupe constitué par les atomes d'oxygène, d'azote et de soufre, R¹, R², R³, identiques ou différents, représentent un radical alkyle en C₁ à C₄,
Y représente un atome d'hydrogène ou un groupe 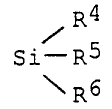 dans lequel R⁴, R⁵ et R⁶, identiques ou différents, représentent un radical alkyle en C₁ à C₄
   et n représente le nombre 1 ou 2.

Le ou les substituants de R peuvent notamment être choisis dans le groupe constitué par les atomes d'halogène, les groupes, halogénés ou non, aromatiques ou hétéroaromatiques et les groupes, halogénés ou non, alkoxy, de préférence en C₁ à C₃, ou aryloxy, par exemple tel que méthoxy, phényloxy, halogénophényloxy. Lorsque R représente un radical cyclique, les substituants peuvent également être choisis parmi les radicaux aliphatiques halogénés ou non et le groupe nitro.

Les atomes d'halogène sont de préférence choisis parmi les atomes de chlore, de brome et de fluor.

Lorsque R représente un radical hétéroaromatique, le ou les hétéroatomes sont plus particulièrement l'azote.

De préférence, R¹, R², R³, R^{4,} R⁵ et R⁶ représentent le radical méthyle.

Parmi les isocyanates d'acyle qui sont obtenus par le procédé selon l'invention, on peut en particulier citer :

Les isocyanates d'acétyle, de trichloroacétyle, de phénylacétyle, de méthacryloyle, de 3-éthoxy ou 3-méthoxyacryloyle, d'hexanoyle, de palmitoyle, de stéaroyle, d'isobutyryle, de pivaloyle, de cinnamoyle, de cyclopentanecarbonyle, de cyclohexanecarbonyle, de benzoyle, de 2,6-difluorobenzoyle, d'o-méthoxybenzoyle, de 2,6-dichlorobenzoyle, de nitrobenzoyle, de naphtoyle, de nicotinoyle, de chloronicotinoyle, de 5-nitrofuroyle, de malonyle, de succinyle, d'adipoyle, de téréphtaloyle et de fumaryle.

Le procédé peut être mis en oeuvre sans solvant ou avec un milieu solvant organique inerte vis à vis des composés. Lorsqu'on souhaite préparer des isocyanates d'acyle qui sont peu stables et sensibles aux conditions réactionnelles telles que la température par exemple, on préfère effectuer la réaction dans un milieu solvant. Parmi les solvants inertes convenant bien, on peut citer les hydrocarbures aliphatiques chlorés ou non, tels que l'hexane, le cyclohexane, l'heptane, le dichlorométhane et le 1,2-dichloroéthane, les hydrocarbures aromatiques tels que le toluène, le chlorobenzène, les di- et trichlorobenzène, les éthers-oxydes tels que l'oxyde de diéthyle, le dioxanne, le tétrahydrofuranne. Les solvants préférés sont les hydrocarbures aliphatiques chlorés.

Le carboxamide silylé et le chlorure d'oxalyle sont généralement mis à réagir en quantité stoéchiométrique, mais on peut également utiliser un excès de chlorure d'oxalyle tel que le rapport molaire chlorure d'oxalyle/carboxamide soit compris entre 1 et 1,5. Le carboxamide est le plus souvent ajouté au chlorure d'oxalyle contenu dans le réacteur.

La température de la réaction est généralement comprise entre - 15°C et + 120°C, et de préférence entre - 15°C et + 100°C. Pendant la phase d'addition du carboxamide, le milieu réactionnel est de préférence maintenu à une basse température, puis la réaction est souvent achevée à une température plus élevée, par exemple lorsqu'un solvant est utilisé, à la température de reflux du solvant.

Lorsque le produit secondaire obtenu est le triméthylchlorosilane, il est enlevé du milieu très facilement compte tenu de son bas point d'ébullition. L'isocyanate peut être ensuite extrait aisément, par exemple par distillation. Lorsque l'isocyanate est peu stable, on ne le sépare pas du solvant et on utilise la solution de l'isocyanate dans le solvant pour effectuer les réactions ultérieures de transformation de l'isocyanate.

Les isocyanates d'acyle sont très utiles pour la préparation de nombreux produits du domaine de la pharmacie, de l'agrochimie et des polymères. On peut citer par exemple l'isocyanate d'acétyle utilisé comme intermédiaire clé dans la préparation de triazolinones. L'isocyanate de trichloroacétyle est très employé pour former des fonctions carbamates au cours de la synthèse d'antibiotiques en particulier de la "céfuroxime". L'isocyanate de méthacryloyle, très réactif, permet par copolymérisation, d'obtenir des matériaux utilisables pour des revêtements réticulables, pour des adhésifs dentaires ou pour des élastomères acryliques. De nouveaux médicaments tels que les inhibiteurs de lipoxygénase sont préparés à partir d'isocyanate de benzoyle. Les isocyanates de benzoyle substitués tels que l'isocyanate de 2,6-difluorobenzoyle sont très utilisés pour fabriquer de nombreuses benzoylurées insecticides.

Les exemples qui suivent sont destinés à illustrer la présente invention sans toutefois la limiter.

### Exemple 1 : Préparation de l'isocyanate d'hexanoyle à partir du N,N-bis(triméthylsilyl)hexanamide.

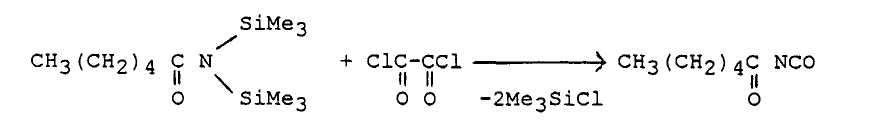

Dans un ballon bicol de 250 ml muni d'un réfrigérant, on introduit une solution de 1,5 g (11,80 mmol) de chlorure d'oxalyle dans 80 ml de 1,2-dichloroéthane (1,2-DCE). On refroidit la solution à O°C par un bain de glace et on ajoute, goutte à goutte en 20 min, sous agitation, 3,3 g (11,6 mmol) de N,N-bis(triméthylsilyl)hexanamide pur. Le mélange réactionnel prend une coloration jaune clair et reste homogène.

On achève la réaction par un chauffage du mélange au reflux du 1,2-DCE pendant 2 heures. On enlève le solvant et le triméthylchlorosilane formé par distillation sous pression normale jusqu'à ce que le volume restant soit d'environ 10 ml puis on effectue une distillation sous pression réduite. On obtient 0,98 g (rendement (Rdt) : 60%) de l'isocyanate attendu qui présente les caractéristiques suivantes :
Liquide incolore très sensible à l'air.
- Point d'ébullition (Eb) :: 40°C/5.10⁻² mm Hg.
- IR(CDCl₃) :: 1730 cm⁻¹ (C=O), 2240 cm⁻¹ (NCO),
2880 cm⁻¹, 2940 cm⁻¹ et 2960 cm⁻¹ (CH).
- RMN ¹H(CDCl₃) :: Massifs complexes centrés sur :
+ 0,9 ppm (m, 3H), + 1,4 ppm (m, 6H), + 2,4 ppm (m, 2H).

### Exemple 2 : Préparation de l'isocyanate d'hexanoyle à partir du N-triméthylsilylhexanamide.

Dans un ballon bicol de 250 ml muni d'un réfrigérant, on introduit une solution de 4,34 g (34,17 mmol) de chlorure d'oxalyle dans 150 ml de 1,2-DCE. On refroidit la solution à 0°C et on ajoute, goutte à goutte en 30 min, sous agitation, une solution de 6,39 g (34,17 mmol) de N-triméthylsilylhexanamide dissous dans 30 ml de 1,2-DCE. Un précipité blanc se forme. Il disparaît dès le retour à la température ambiante.

On achève la réaction par chauffage du mélange réactionnel au reflux du solvant pendant 1 heure. Après retour à la température ambiante, on obtient une solution limpide de couleur jaune clair.

On enlève le solvant et le triméthylchlorosilane formé par distillation sous pression normale jusqu'à ce que le volume résiduel soit d'environ 10 ml, puis on effectue une distillation sous pression réduite. On obtient 2,70 g (Rdt : 56%) de l'isocyanate attendu ayant les caractéristiques suivantes :
- Eb :: 69-70°C/20 mm Hg.
- IR(CDCl₃) :: 1740 cm⁻¹ (CO), 2250 cm⁻¹ (NCO),
2900 cm⁻¹ (CH).

### Exemple 3 : Préparation de l'isocyanate de benzoyle à partir du N,N-bis(triméthylsilyl)benzamide.

Dans un ballon bicol de 250 ml muni d'un réfrigérant, on introduit une solution de 1,27 g (10 mmol) de chlorure d'oxalyle dans 40 ml de 1,2-DCE. On la refroidit à 0°C et on ajoute, goutte à goutte en 15 min, 2,47 g (9,3 mmol) de N,N-bis(triméthylsilyl)benzamide pur. La coloration du mélange réactionnel devient jaune et il se forme un précipité blanc. Après un chauffage du milieu réactionnel d'une heure au reflux du 1,2-DCE, l'amide disilylé de départ est totalement consommé (contrôle IR). La solution est limpide et de couleur brune.

On recueille le solvant et le triméthylchlorosilane formé dans un piège à -196°C par évaporation sous vide (40°C/ 50 mm Hg). Lorsque le volume résiduel est d'environ 3 ml, on transvase le produit brut dans un ballon de distillation et on effectue une distillation sous pression réduite. On obtient 1,03 g (Rdt : 75%) d'isocyanate de benzoyle ayant les caractéristiques suivantes :
Liquide incolore sensible à l'air.
- Eb :: 125°C/50 mm Hg.
- IR(1,2-DCE) :: 1600 cm⁻¹ (aromatique),
1700 cm⁻¹ (CO), 2250 cm⁻¹ (NCO).
- RMN ¹³C (CDCl₃) :: + 128,688 ppm (s, C méta),
+ 130,398 ppm (s, C ortho),
+ 134,542 ppm (s, C para),
+ 164,884 ppm (s, C = O).

Le triméthylchlorosilane récupéré est dosé par RMN ¹H par rapport à du benzène pris comme référence. Le rendement est de 84,3% par rapport à la quantité théorique attendue.

### Exemple 4 : Préparation de l'isocyanate de benzoyle à partir du N-triméthylsilylbenzamide.

Dans un ballon bicol de 250 ml muni d'un réfrigérant, on introduit une solution de 2,54 g (20 mmol) de chlorure d'oxalyle dans 90 ml de 1,2-DCE. On la refroidit à 0°C et on ajoute, goutte à goutte en 30 min, une solution de 3,86 g (20 mmol) de N-triméthylsilylbenzamide dissous dans 30 ml de 1,2-DCE. Il se forme un précipité blanc qui disparait lorsque le mélange réactionnel revient à la température ambiante. On achève la réaction par un chauffage du mélange à reflux du 1,2-DCE pendant une heure. La coloration de la solution est jaune clair, et il n'y a pas de précipité.

On opère ensuite comme à l'exemple précédent. Le solvant et le triméthylchlorosilane formé sont recueillis. Puis on distille, sous pression réduite, le liquide marron restant. On obtient 1,76 g (Rdt : 60%) d'isocyanate de benzoyle ayant les caractéristiques suivantes :
Liquide jaune pale, très sensible à l'air.
- Eb :: 95-98°C/20 mm Hg.
- IR(CDCl₃) :: 1600 cm⁻¹ (aromatique),
1700 cm⁻¹ (CO),
2250 cm⁻¹ (NCO).

### Exemple 5 : Préparation de l'isocyanate de 2,6-difluorobenzoyle.

Dans un ballon de 50 ml, on introduit une solution de 0,46 g (3,6 mmol) de chlorure d'oxalyle dans 20 ml de 1,2-DCE. On ajoute, goutte à goutte, sous agitation, 1 g (3,3 mmol) de N,N-bis(triméthylsilyl)-2,6-difluoro-benzamide pur. La solution devient jaune. La réaction est immédiate. Par spectrométrie IR, on a identifié l'isocyanate formé et le chlorure d'oxalyle résiduel. Les produits légers sont évaporés sous 0,8 mm Hg à la température ambiante.

On obtient 0,40 g (Rdt : 67%) de l'isocyanate attendu par distillation à 36°C/0,8 mm Hg.

### Exemple 6 : Préparation de l'isocyanate d'acétyle.

Dans un ballon de 100 ml muni d'un réfrigérant, on introduit une solution de 0,62 g (4,88 mmol) de chlorure d'oxalyle dans 20 ml de 1,2-DCE. On la refroidit à 0°C et on additionne, goutte à goutte, en 10 min, 1 g (4,88 mmol) de N,N-bis(triméthylsilyl)acétamide. Le mélange devient jaune clair et se trouble légèrement. On achève la réaction par chauffage du mélange au reflux du 1,2-DCE pendant 1 heure. La coloration du mélange réactionnel devient brun foncé.

On ne sépare pas l'isocyanate d'acétyle obtenu du solvant mais on le transforme en carbamate, en ajoutant dans le milieu, 0,36 g (4,88 mmol) de tertiobutanol. On obtient 0,39 g (Rdt : 50%) de CH₃-C(O)-NH-C(O)-O-C(CH₃)₃ caractérisé par analyse RMN ¹H et IR.

### Exemple 7 : Préparation de l'isocyanate de nicotinoyle

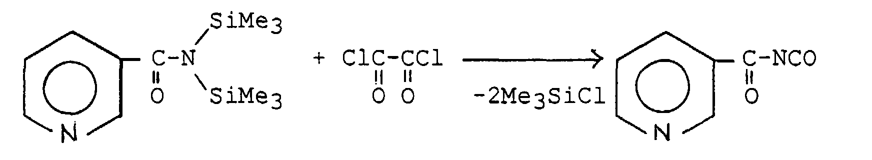

Dans un ballon bicol de 250 ml muni d'un réfrigérant, on introduit une solution de 1,91 g (15 mmol) de chlorure d'oxalyle dans 100 ml de 1,2-DCE. On la refroidit à 0°C et on ajoute, goutte à goutte en 15 min, 3,99 g (15 mmol) de N,N-bis(triméthylsilyl)nicotinamide pur. Il se forme un précipité blanc et la solution se colore en jaune clair. On chauffe le mélange réactionnel au reflux du 1,2-DCE pendant une heure.

On ajoute dans le mélange réactionnel, la quantité stoechiométrique, par rapport au nicotinamide silylé de départ, de tertiobutanol et on obtient le carbamate 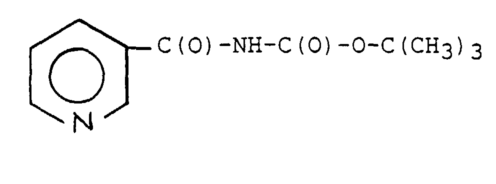 caractérisé par analyse RMN ¹H et IR, avec un rendement de 30%.

### Exemple 8 : Préparation de l'isocyanate de 2-chloronicotinoyle.

Dans un ballon bicol de 250 ml muni d'un réfrigérant, on introduit une solution de 1,91 g (15 mmol) de chlorure d'oxalyle dans 100 ml de 1,2-DCE. On la refroidit à 0°C et on ajoute, goutte à goutte en 15 min, 4,50 g (15 mmol) de N,N-bis(triméthylsilyl)2-chloronicotinamide. La solution prend une coloration jaune clair. Après 15 min à 0°C, l'analyse IR effectuée sur un prélèvement du milieu réactionnel montre la formation importante de l'isocyanate : bande CO à 1720 cm⁻¹ et bande NCO à 2250 cm⁻¹.

On ajoute dans le milieu réactionnel, la quantité stoechiométrique, par rapport au chloronicotinamide silylé de départ, de tertiobutanol. On obtient le carbamate 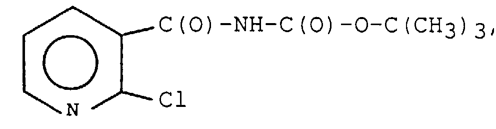 caractérisé par analyse RMN ¹H et IR, avec un rendement de 96%.

### Exemple 9 : Préparation de l'isocyanate de méthacryloyle.

Dans un ballon bicol de 500 ml muni d'un réfrigérant, on introduit, sous atmosphère d'azote, 29,8 g (130 mmol) de N,N-bis(triméthylsilyl)méthacrylamide dans 250 ml de 1,2-DCE. On ajoute goutte à goutte, sous agitation et à -12°C, 16,5 g de chlorure d'oxalyle dilué dans 20 ml de 1,2-DCE. Après retour à la température ambiante, le mélange est chauffé au reflux du solvant pendant 1 heure. La coloration du mélange devient brune. Les produits volatils sont transférés sous vide (10⁻¹ mm Hg) et piégés à -196°C. La solution obtenue est incolore. Une première distillation très lente sous pression atmosphérique permet de recueillir le triméthylchlorosilane (Eb : 60°C, Rdt : 93%) et d'éliminer une partie du DCE. La distillation est arrêtée lorsque le volume résiduel est de 50 ml. On termine la distillation avec un appareil de taille appropriée. On obtient 4,8 g (Rdt : 17%) de l'isocyanate attendu ayant les caractéristiques suivantes :
Liquide incolore, sensible à l'air qui jaunit lorsqu'il est conservé à température ambiante.
- IR(CDCl₃) :: 2240 cm⁻¹ (CO) bande forte,
1700 cm⁻¹ (CO) bande forte,
1645 cm⁻¹ (C = C) bande fine.

## Revendications

1. Procédé de préparation d'isocyanates d'acyle, caractérisé en ce qu'on fait réagir le chlorure d'oxalyle avec un N-trialkylsilylcarboxamide ou un N,N-bis(trialkylsilyl)carboxamide.

2. Procédé selon la revendication 1, caractérisé en ce que les carboxamides sont représentés par la formule générale : 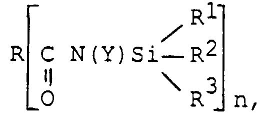 dans laquelle :
R représente un radical - aliphatique linéaire ou ramifié, en C₁ à C₂₀, saturé ou non, substitué ou non,
- cycloaliphatique en C₄ à C₇, saturé ou non, substitué ou non,
- phényle, phénylène, naphtyle ou naphtylène, substitué ou non,
- hétéroaromatique, de préférence à 5 ou 6 chaînons, substitué ou non,
R¹, R² et R³, identiques ou différents, représentent un radical alkyle en C₁ à C₄,
Y représente un atome d'hydrogène ou un groupe 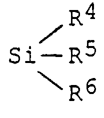 dans lequel R⁴, R⁵ et R⁶, identiques ou différents, représentent un radical alkyle en C₁ à C₄ et n représente le nombre 1 ou 2.

3. Procédé selon la revendication 2, caractérisé en ce que, lorsque R représente un radical hétéroaromatique, le ou les hétéroatomes sont choisis dans le groupe constitué par les atomes d'oxygène, d'azote et de soufre.

4. Procédé selon la revendication 2, caractérisé en ce que R¹, R², R³, R⁴, R⁵ et R⁶ représente le radical méthyle.

5. Procédé selon la revendication 1, caractérisé en ce que la réaction est effectuée dans un milieu solvant organique inerte.

6. Procédé selon la revendication 5, caractérisé en ce que le solvant est choisi parmi les hydrocarbures aliphatiques chlorés ou non, les hydrocarbures aromatiques chlorés ou non et les éthers-oxydes.

7. Procédé selon la revendication 6, caractérisé en ce que le solvant est choisi parmi les hydrocarbures aliphatiques chlorés.

8. Procédé selon la revendication 1, caractérisé en ce que la température de réaction est comprise entre - 15°C et + 120°C.

## Patentansprüche

1. Verfahren zur Herstellung von Acylisocyanaten, dadurch gekennzeichnet, daß man Oxalylchlorid mit einem N-Trialkylsilylcarboxamid oder einem N,N-Bis-(trialkylsilyl)-carboxamid umsetzt.

2. Verfahren nach Anspruch 1, dadurch gekennzeichnet, daß die Carboxamide die folgende allgemeine Formel aufweisen 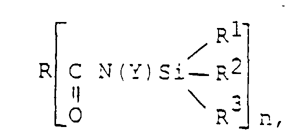
in der R einen aliphatischen, linearen oder verzweigten, gesättigten oder ungesättigten, substituierten oder unsubstituierten C₁- bis C₂₀-Rest,
- einen cycloaliphatischen, gesättigten oder ungesättigten, substituierten oder unsubstituierten C₄- bis C₇-Rest,
- einen gesättigten oder ungesättigten Phenyl-, Phenylen-, Naphthyl- oder Napthylenrest oder
- einen substituierten oder unsubstituierten heteroaromatischen Rest mit vorzugweise 5 oder 6 Ringgliedern bedeutet,
R¹, R² und R³, die gleich oder verschieden sind, jeweils einen C₁- bis C₄-Alkylrest bedeuten,
Y ein Wasserstoffatom oder eine Gruppe der Formel 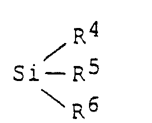 bedeutet, in der R⁴, R⁵ und R⁶, die gleich oder verschieden sind, jeweils einen C₁- bis C₄-Alkylrest bedeuten und
n eine Zahl mit einem Wert von 1 oder 2 bedeutet.

3. Verfahren nach Anspruch 2, dadurch gekennzeichnet, daß dann, wenn R einen heteroaromatischen Rest bedeutet, das oder die Heteroatome unter Sauerstoff-, Stickstoff- und Schwefelatomen ausgewählt sind.

4. Verfahren nach Anspruch 2, dadurch gekennzeichnet, daß R¹, R², R³, R⁴, R⁵ und R⁶ jeweils einen Methylrest bedeuten.

5. Verfahren nach Anspruch 1, dadurch gekennzeichnet, daß die Umsetzung in einem inerten organischen Lösungsmittel durchgeführt wird.

6. Verfahren nach Anspruch 5, dadurch gekennzeichnet, daß das Lösungsmittel unter chlorierten oder unchlorierten, aliphatischen Kohlenwasserstoffen, chlorierten oder unchlorierten, aromatischen Kohlenwasserstoffen und Etheroxiden ausgewählt ist.

7. Verfahren nach Anspruch 6, dadurch gekennzeichnet, daß das Lösungsmittel unter chlorierten aliphatischen Kohlenwasserstoffen ausgewählt ist.

8. Verfahren nach Anspruch 1, dadurch gekennzeichnet, daß die Umsetzungstemperatur im Bereich von -15°C bis +120°C liegt.

## Claims

1. Method for preparing acyl isocyanates, characterized in that oxalyl chloride is reacted with an N-trialkylsilylcarboxamide or an N,N-bis(trialkylsilyl)carboxamide.

2. Method according to claim 1, characterized in that the carboxamides are represented by the general formula: 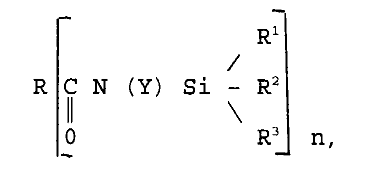 in which:
R represents
- a substituted or unsubstituted, saturated or unsaturated, linear or branched aliphatic radical with C₁ to C₂₀,
- a substituted or unsubstituted, saturated or unsaturated cycloaliphatic radical with C₄ to C₇,
- a substituted or unsubstituted phenyl, phenylene, naphthyl or naphthalene radical,
- a substituted or unsubstituted heteroaromatic, preferably with 5 or 6 members,
R¹, R² and R³, either identical or different, represent an alkyl radical with C₁ to C₄,
Y represents a hydrogen atom or ar 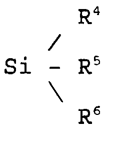 group in which R⁴, R⁵ and R⁶, either identical or different, represent an alkyl radical with C₁ to C₄ and
n represents the number 1 or 2.

3. Method according to claim 2, characterized in that, when R represents an heteroaromatic radical, the heteroatom(s) are chosen from the group consisting of oxygen, nitrogen and sulfur atoms.

4. Method according to claim 2, characterized in that R¹, R², R³, R⁴, R⁵ and R⁶ represent the methyl radical.

5. Method according to claim 1, characterized in that the reaction is carried out in an inert organic solvent medium.

6. Method according to claim 5, characterized in that the solvent is chosen from among chlorinated or unchlorinated aliphatic hydrocarbons, chlorinated or unchlorinated aromatic hydrocarbons and ether-oxides.

7. Method according to claim 6, characterized in that the solvent is chosen from among chlorinated aliphatic hydrocarbons.

8. Method according to claim 1, characterized in that the reaction temperature is comprised between -15°C and +120°C.
